(19) Europäisches Patentamt
European Patent Office
Office européen des brevets

(11) Numéro de publication: **0 577 470 B1**

(12) **FASCICULE DE BREVET EUROPEEN**

(45) Date de publication de fascicule du brevet: **24.05.95**

(51) Int. Cl.$^{6}$: **C07D 239/42**, C07D 401/14, A61K 31/505

(21) Numéro de dépôt: **93401594.2**

(22) Date de dépôt: **22.06.93**

(54) **Dérivés de 2-amino-N-[[[4-(aminocarbonyl)pyrimidin-2-yl]-amino]alkyl]pyrimidine-4-carboxamide, leur préparation et leur application en thérapeutique.**

(30) Priorité: **03.07.92 FR 9208199**
**14.12.92 FR 9215037**

(43) Date de publication de la demande:
**05.01.94 Bulletin 94/01**

(45) Mention de la délivrance du brevet:
**24.05.95 Bulletin 95/21**

(84) Etats contractants désignés:
**AT BE CH DE DK ES FR GB GR IE IT LI LU MC NL PT SE**

(56) Documents cités:
**EP-A- 0 416 841**
**EP-A- 0 435 749**
**EP-A- 0 480 794**
**EP-A- 0 511 072**
**EP-A- 0 520 883**
**US-A- 5 075 308**

(73) Titulaire: **SYNTHELABO**
**22, Avenue Galilée**
**F-92350 Le Plessis Robinson (FR)**

(72) Inventeur: **George, Pascal**
**19 rue des Ouatre Vents**
**F-78730 Saint Arnoult en Yvelines (FR)**
Inventeur: **Marabout, Benoit**
**6 rue Georges Ritz**
**F-91300 Massy (FR)**
Inventeur: **Froissant, Jacques**
**Cidex 981 Brevainville**
**F-41160 Morée (FR)**
Inventeur: **Merly, Jean-Pierre**
**11 avenue Jules Guesde**
**F-92330 Sceaux (FR)**

(74) Mandataire: **Ludwig, Jacques et al**
**SYNTHELABO,**
**Service Brevets,**
**B.P. 72**
**F-92352 Le Plessis Robinson Cédex (FR)**

EP 0 577 470 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés de 2-amino-*N*-[[[4-(aminocarbonyl)pyrimidin-2-yl]amino]-alkyl]pyrimidine-4-carboxamide, leur préparation et leur application en thérapeutique.

Les composés de l'invention répondent à la formule générale (I)

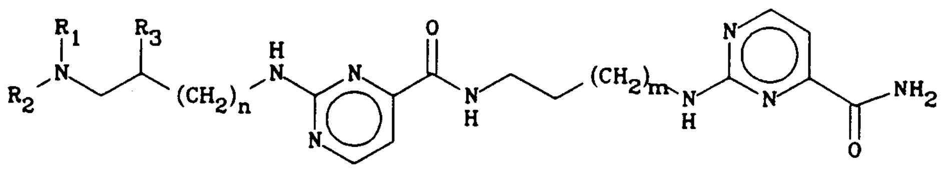

(I)

dans laquelle
n représente le nombre 0 ou 1,
m représente le nombre 0 ou 1,
$R_1$ représente un groupe méthyle, auquel cas
$R_2$ représente un groupe phénoxy($C_1$-$C_4$)alkyle (dont le groupe phénoxy porte éventuellement 1 ou 2 substituants choisis parmi les atomes d'halogènes et les groupes méthoxy), ou bien $R_1$ et $R_2$ forment ensemble, et avec l'atome d'azote qui les porte, un groupe 4-phénoxypipéridin-1-yle (dont le groupe phénoxy porte éventuellement 1 ou 2 substituants choisis parmi les atomes d'halogènes et les groupes méthoxy), un groupe phénoxyméthylpipéridin-1-yle (dont le groupe phénoxy porte éventuellement 1 ou 2 groupes alkyles en $C_1$-$C_4$), ou un groupe 4-phénylpipérazin-1-yle (dont le groupe phényle porte éventuellement 1 ou 2 substituants choisis parmi les atomes d'halogènes et les groupes méthoxy), et
$R_3$ représente un atome d'hydrogène ou, et seulement lorsque n est égal à 1, un groupe hydroxy ou un groupe méthoxy.

Les composés de l'invention peuvent exister à l'état de bases ou de sels d'addition à des acides. Par ailleurs, lorsque la molécule contient un atome de carbone asymétrique, un composé peut exister sous forme optiquement pure ou sous forme de mélange d'isomères optiques.

Conformément à l'invention, on peut préparer les composés de formule générale (I) par un procédé illustré par le schéma qui suit.

## Schéma

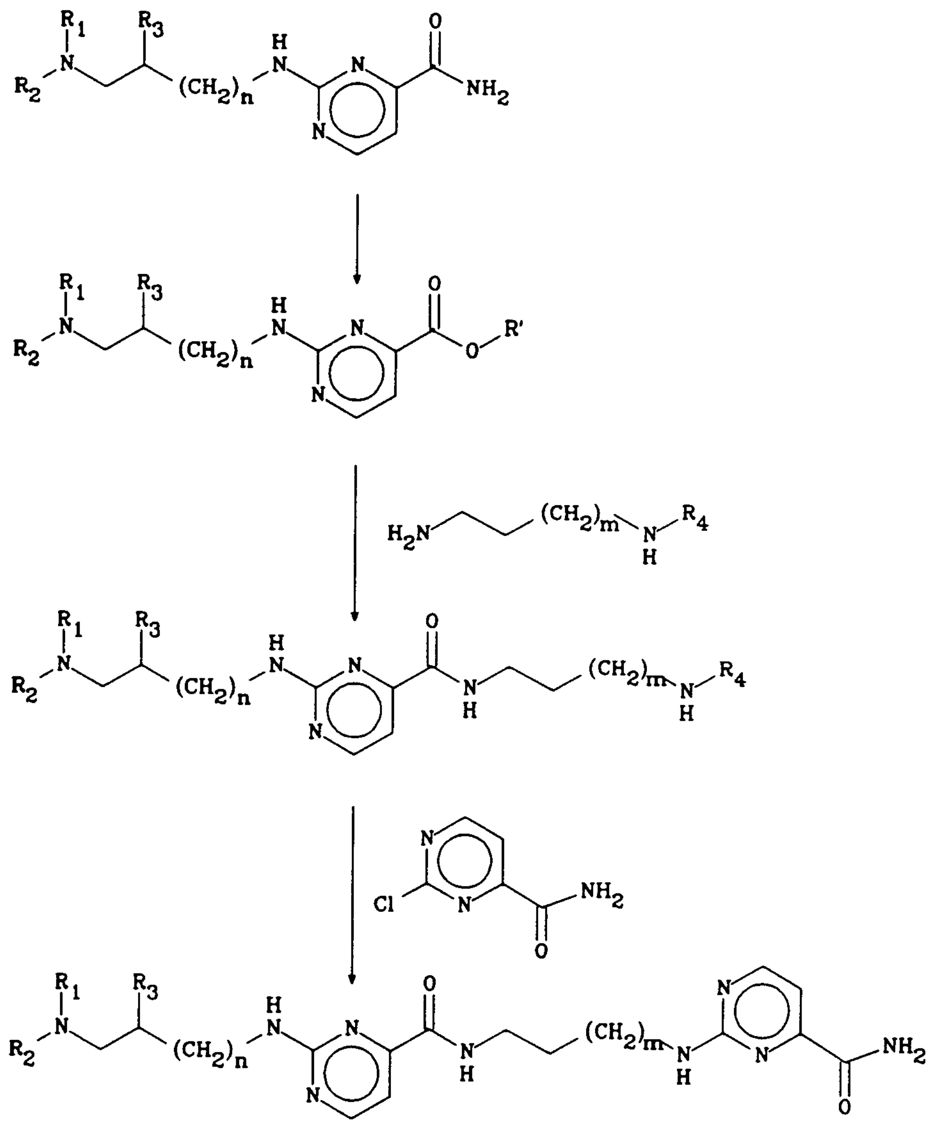

(II)

(III)

(IV)

(V)

(VI)

(I)

On transforme un amide de formule générale (II) (dans laquelle quelle n, $R_1$, $R_2$ et $R_3$ sont tels que définis ci-dessus) en un ester de formule générale (III) (dans laquelle R' représente un groupe alkyle en $C_1$-$C_4$), par réaction avec un alcool aliphatique en $C_1$-$C_4$, par exemple le méthanol, en présence d'un acide, par exemple l'acide chlorhydrique gazeux, à une température de 0 à 100 °C.

Ensuite on transforme l'ester ainsi obtenu, par réaction avec une amine de formule générale (IV) (dans laquelle m est tel que défini ci-dessus et $R_4$ représente soit un atome d'hydrogène, soit un groupement protecteur d'amine, par exemple un groupe tertiobutyloxycarbonyle) en amide de formule générale (V), dans un alcool aliphatique comme solvant, par exemple le méthanol, à une température de 0 à 60 °C.

Ensuite, si nécessaire, on déprotège l'amine de formule générale (V) par une méthode connue, par exemple par traitement avec l'acide trifluoroacétique dans le dichlorométhane lorsque $R_4$ représente un groupe tertiobutyloxycarbonyle.

Et enfin on fait réagir l'amine de formule générale (V) (dans laquelle $R_4$ représente un atome d'hydrogène) avec le 2-chloropyrimidine-4-carboxamide de formule (VI) dans un solvant aprotique, par exemple le *N,N*-diméthylformamide, en présence d'une base, par exemple le carbonate de potassium, à une température de 20 à 40 °C, pour obtenir le composé de formule générale (I).

Les dérivés de 2-aminopyrimidine-4-carboxamide de formule générale (II) peuvent être obtenus par des méthodes analogues à celles décrites dans les demandes de brevets FR-2675799, FR-2678271 et EP-0480794.

Les diamines monoprotégées de formule générale (IV) peuvent être préparées par des méthodes analogues à celles décrites dans *Synthesis* (1984) 1032-1033 et *Synthesis* (1990) 366-368.

Les exemples suivants illustrent en détail la préparation de quelques composés selon l'invention. Les microanalyses élémentaires et les spectres IR et RMN confirment les structures des composés obtenus. Les numéros des composés, indiqués entre parenthèses dans les titres, correspondent à ceux du tableau donné plus loin.

Exemple 1 (Composé n° 4).

Chlorhydrate de *N*-[2-[[4-(aminocarbonyl)pyrimidin-2-yl]amino]éthyl]-2-[[3-[4-(5-chloro-2-méthoxyphényl)-pipérazin-1-yl]propyl]amino]pyrimidine-4-carboxamide.

1.1. 2-[[3-[4-(5-chloro-2-méthoxyphényl)pipérazin-1-yl]propyl]amino]pyrimidine-4-carboxylate de méthyle.

Dans un ballon de 0,5 l, on introduit 7,8 g (19,2 mmoles) de 2-[[3-[4-(5-chloro-2-méthoxyphényl)-pipérazin-1-yl]propyl]amino]pyrimidine-4-carboxamide dans 300 ml de méthanol, puis on fait passer un courant d'acide chlorhydrique gazeux pendant quelques minutes et on chauffe le mélange à la température du reflux du méthanol pendant 1 h 45. On évapore le solvant sous pression réduite, on ajoute au résidu 200 ml de dichlorométhane puis on refroidit à 0 °C. On alcalinise le mélange avec une solution aqueuse saturée en hydrogénocarbonate de sodium.

On sèche la phase organique sur sulfate de sodium, filtre puis évapore le solvant sous pression réduite. On purifie le résidu par chromatograhie sur colonne de gel de silice (éluant : mélange dichlorométhane/méthanol 100/0 à 90/10) puis recristallisation dans du cyclohexane. On isole 5,84 g (13,9 mmoles) d'ester.

Point du fusion : 118,5-119 °C.

1.2. [2-[[[2-[[3-[4-(5-Chloro-2-méthoxyphényl)pipérazin-1-yl]propyl]amino]pyrimidin-4-yl]carbonyl]amino]-éthyl] carbamate de 1,1-diméthyléthyle.

Dans un ballon de 500 ml, on introduit 5,07 g (31,65 mmoles) de (2-aminoéthyl)carbamate de 1,1-diméthyléthyle, 10 g (23,8 mmoles) de 2-[[3-[4-(5-chloro-2-méthoxyphényl)pipérazin-1-yl]propyl]amino]-pyrimidine-4-carboxylate de méthyle dans 50 ml de propan-2-ol et on chauffe le mélange au reflux pendant 14 h. On évapore le solvant sous pression réduite et on purifie le résidu par chromatograhie sur colonne de gel de silice (éluant dichlorométhane/méthanol 100/0 à 90/10) pour obtenir 10,77 g (19,65 mmoles) de composé sous forme d'huile, qu'on utilise tel quel dans l'étape suivante.

1.3. *N*-(2-Aminoéthyl)-2-[[3-[4-(5-chloro-2-méthoxyphényl)pi-pérazin-1-yl)propyl]amino]pyrimidine-4-carboxamide.

Dans un ballon de 0,5 l, on introduit 10,77 g (19,65 mmoles) de composé [2-[[[2-[[3-[4-(5-Chloro-2-méthoxyphényl)pipéra-zin-1-yl]propyl]amino]pyrimidin-4-yl]carbonyl]amino]éthyl]-carbamate de 1,1-diméthyléthyle dans 50 ml d'eau puis, goutte à goutte, 25 ml d'acide chlorhydrique concentré. On refroidit le mélange à 0 °C avec un mélange glace/sel/eau, puis on additionne, par portions, de la soude à 30% jusqu'à pH basique.

On extrait au dichlorométhane, on sèche la phase organique sur sulfate de sodium, on filtre et évapore les solvants sous pression réduite, et on obtient 8,8 g (19,65 mmoles) d'huile que l'on utilise telle quelle dans l'étape suivante.

1.4. Chlorhydrate de *N*-[2-[[4-(aminocarbonyl)pyrimidin-2 yl]amino]éthyl]-2-[[3-[4-(5-chloro-2-méthoxyphényl)-pipérazin-1-yl]propyl] amino]pyrimidine-4-carboxamide.

Dans un ballon de 0,5 l sous atmosphère d'argon, on introduit 8,8 g (19,65 mmoles) de *N*-(2-Aminoéthyl)-2-[[3-[4-(5-chloro-2-méthoxyphényl)pipérazin-1-yl]propyl]amino]pyrimidine-4-carboxamide, 3,1 g (19,7 mmoles) de 2-chloropyrimidine-4-carboxamide, 4,0 g (29 mmoles) de carbonate de potassium dans 250 ml d'acétonitrile, et on chauffe au ref lux pendant 18 h.

On refroidit le mélange à température ambiante, on collecte l'insoluble par filtration, puis on le lave avec de l'eau. On dissout le produit obtenu dans un mélange dichlorométhane/méthanol, puis on le purifie par chromatograhie sur colonne de gel de silice (éluant dichlorométhane/méthanol 100/0 à 85/15). On isole, après recristallisation dans un mélange acétonitrile/dichlorométhane, 7,03 g (12,35 mmoles) de composé à l'état de base. Point de fusion : 196-199 °C.

Pour préparer le chlorhydrate on en dissout 3,03 g (5,32 mmoles) dans un mélange de 50 ml de dichlorométhane et 50 ml de méthanol, et on ajoute 53,2 ml d'acide chlorhydrique 0,1N dans le propan-2-ol. On évapore les solvants sous pression réduite, et on recristallise le résidu dans un mélange de méthanol et d'acétate d'éthyle. On obtient finalement 2,63 g de chlorhydrate.

Point de fusion : 197,5-200,5 °C.

Exemple 2 (Composé n° 11).

*N*-[2-[[4-(Aminocarbonyl)pyrimidin-2-yl]amino]éthyl)-2-[[2-[4-(2,5-diméthoxyphényl)pipérazin-1-yl]éthyl]-amino]pyrimidine-4-carboxamide.

2.1. 2-[[2-[4-(2,5-Diméthoxyphényl)pipérazin-1-yl)éthyl)ami-no] pyrimidine-4-carboxylate de méthyle.
Dans un ballon de 1 l, on introduit 8,76 g (22,66 mmoles) de 2-[[2-[4-(2,5-diméthoxyphényl)pipérazin-1-yl]éthyl]amino]pyrimidine-4-carboxamide dans 650 ml de méthanol, puis on fait passer un courant d'acide chlorhydrique gazeux pendant quelques minutes et on chauffe le mélange à la température du reflux du méthanol pendant 5 h. On évapore le solvant sous pression réduite, on ajoute au résidu 300 ml de dichlorométhane puis on alcalinise le mélange avec une solution aqueuse saturée en hydrogénocarbonate de sodium. On sèche la phase organique sur sulfate de sodium, filtre puis évapore le solvant sous pression réduite. Après purification par chromatographie sur colonne de gel de silice (éluant : mélange dichlorométhane/méthanol 100/0 à 90/10) puis recristallisation dans le cyclohexane on isole 6,93 g (17,26 mmoles) d'ester.
Point de fusion : 85,5-87 °C.
2.2. *N*-(2-Aminoéthyl)-2-[[2-[4-(2,5-diméthoxyphényl)pipérazin-1-yl]ethyl]amino]pyrimidine-4-carboxamide.
Dans un ballon de 0,25 l, on introduit 1,5 g (3,74 mmoles) de 2-[[2-[4-(2,5-diméthoxyphényl)pipérazin-1-yl]éthyl]amino]pyrimidine-4-carboxylate de méthyle dans 20 ml de dichlorométhane et 150 ml de méthanol, puis 3 ml d'éthylènediamine (2,7 g, 44,9 mmoles).
On agite le mélange pendant 4 h à température ambiante puis on évapore les solvants sous pression réduite.
On dissout le résidu brut dans 150 ml de dichlorométhane, et on lave la phase organique cinq fois à l'eau. On sèche sur sulfate de sodium, filtre et évapore le solvant sous pression réduite, et on obtient 1,6 g (3,74 mmoles) d'huile qu'on utilise telle quelle dans l'étape suivante.
2.3. *N*-[2-[[4-(Aminocarbonyl)pyrimidin-2-yl]amino]éthyl]-2-[[2-[4-(2,5-diméthoxyphényl)pipérazin-1-yl]-éthyl]amino]pyrimidine-4-carboxamide.
Dans un ballon de 0,5 l, on introduit 1,6 g (3,74 mmoles) *N*-(2-Aminoéthyl)-2-[[2-[4-(2,5-diméthoxyphényl)pipérazin-1-yl]éthyl]amino]pyrimidine-4-carboxamide, 0,59 g (3,74 mmoles) de 2-chloropyrimidine-4-carboxamide et 0,8 g (5,8 mmoles) de carbonate de potassium dans 200 ml d'acétonitrile et on chauffe le mélange au reflux pendant 16 h.
On évapore le solvant sous pression réduite et on ajoute au résidu brut 200 ml d'eau et 500 ml de dichlorométhane. On sèche la phase organique sur sulfate de sodium puis on évapore le solvant sous pression réduite, et on purifie le résidu par chromatographie sur colonne de gel de silice (éluant : mélange dichlorométhane/méthanol 100/0 à 90/10), puis par recristallisation dans un mélange acétonitrile/dichlorométhane. On obtient 1,1 g (2 mmoles) de composé.
Point de fusion : 158-160 °C.

Exemple 3 (Composé n° 12)

*N*-[2-[[4-(aminocarbonyl)pyrimidin-2-yl]amino]éthyl]-2-[[3-[[2-(2-méthoxyphénoxy)éthyl]méthylamino]propyl]-amino]pyrimidine-4-carboxamide

3.1 2-[[3-[[2-(2-Méthoxyphénoxy)éthyl]méthylamino]propyl]amino]pyrimidine-4-carboxylate de méthyle.
Dans un ballon de 0,5 l on introduit 12,7 g (35,3 mmoles) de 2-[[3-[[2-(2-méthoxyphénoxy)éthyl]-méthylamino]propyl]amino]pyrimidine-4-carboxamide et 300 ml de méthanol, puis on fait passer un courant d'acide chlorydrique gazeux pendant quelques minutes et on chauffe à la température du reflux du méthanol pendant 4h.
On évapore le solvant sous pression réduite, on ajoute au résidu 100 ml de dichlorométhane et on refroidit à 0 °C. On alcalinise le mélange avec une solution aqueuse saturée d'hydrogénocarbonate de sodium, on décante et sèche la phase organique sur sulfate de sodium, on filtre puis on évapore le solvant sous pression réduite.
Après purification par chromatographie sur colonne de gel de silice (éluant : mélange dichlorométhane/méthanol, 96/4 à 88/12), on isole 8,7 g de composé sous forme huileuse qu'on utilise tel quel dans l'étape suivante.
3.2 *N*-(2-aminoéthyl)-2-[[3-[[2-(2-méthoxyphénoxy)éthyl]mé-thylamino]propyl]amino]pyrimidine-4-carboxamide.
Dans un ballon tricol de 0,25 l, on introduit 3,0 g (8 mmoles) de 2-[[3-[[2-(2-méthoxyphénoxy)éthyl]-

méthylamino]propyl]amino]pyrimidine-4-carboxylate de méthyle et 4,8 g (80 mmoles) d'éthylènediamine dans 100 ml d'un mélange 1/1 de méthanol/dichlorométhane. On agite le milieu réactionnel pendant 48h à température ambiante, puis on évapore les solvants sous pression réduite.

On reprend le résidu brut dans 100 ml de dichlorométhane, on lave la phase organique à l'eau (2x100 ml), on sèche sur sulfate de sodium, on filtre, puis on évapore le solvant sous pression réduite pour obtenir 3,05 g de composé sous forme huileuse, qu'on utilise tel quel dans l'étape suivante.

3.3 *N*-[2-[[4-(aminocarbonyl)pyrimidin-2-yl]amino]éthyl]-2-[[3-[[2-(2-méthoxyphénoxy)éthyl]méthylamino]-propyl]amino]pyrimidine-4-carboxamide.

Dans un ballon tricol de 0,25 l, on introduit 3,0 g (7,45 mmoles) de *N*-(2-aminoethyl)-2-[[3-[[2-(2-méthoxyphénoxy)éthyl]méthylamino]propyl]amino]pyrimidine-4-carboxamide, 1,23 g (7,8 mmoles) de 2-chloropyrimidine-4-carboxamide, 1,55 g (11,2 mmoles) de carbonate de potassium, 0,1 g d'iodure de sodium dans 40 ml de *N,N*-diméthylformamide, et on chauffe le milieu réactionnel à 60 ° C pendant 15h.

On refroidit à température ambiante, on verse le produit réactionnel sur 100 ml d'eau et on extrait à l'acétate d'éthyle (3x100 ml). On lave la phase organique à l'eau, on la sèche sur sulfate de sodium, on filtre et on évapore les solvants sous pression réduite.

Après recristallisation dans l'acétate d'éthyle on obtient 1,95 g de composé.

Point de fusion : 131-133 ° C.

Exemple 4 (Composé n ° 13)

*N*-[2-[[4-(aminocarbonyl)pyrimidin-2-yl]amino]éthyl]-2-[[3-[4-[[5-méthyl-2-(1-méthyléthyl)phénoxy]méthyl]-pipéridin-1-yl]propyl]amino]pyrimidine-4-carboxamide.

4.1 2-[[3-[4-[[5-Méthyl-2-(1-méthyléthyl)phénoxy]méthyl]pipéridin-1-yl]propyl]amino]pyrimidine-4-carboxylate de méthyle.

Dans un ballon de 1 l, on introduit 6,3 g (14,8 mmoles) de 2-[[3-[4-[[5-méthyl-2-(1-méthyléthyl)phénoxy]-méthyl]pipéridin-1-yl]propyl]amino]pyrimidine-4-carboxamide et 250 ml de méthanol, puis on fait passer un courant d'acide chlorhydrique gazeux pendant quelques minutes et on chauffe à la température du reflux du méthanol pendant 1h30.

On évapore le solvant sous pression réduite, on ajoute au résidu 150 ml de dichlorométhane et on refroidit à O ° C. On alcanilise le mélange avec une solution aqueuse saturée d'hydrogénocarbonate de sodium, on décante et sèche la phase organique sur sulfate de magnésium, on filtre puis on évapore le solvant sous pression réduite.

Après purification par chromatographie sur colonne de gel de silice (éluant : mélange dichlorométhane/méthanol, 100/0 à 90/10), on isole 4,3 g de composé sous forme huileuse, qu'on utilise tel quel dans l'étape suivante.

4.2 [2-[[[2-[[3-[4-[[5-Méthyl-2-(1-méthyléthyl)phénoxy]méthyl]pipéridin-1-yl]propyl]amino]pyrimidin-4-yl]-carbonyl]amino]éthyl]carbamate de 1,1-diméthyléthyle.

Dans un ballon de 0,1 l, on introduit 4,3 g (9,76 mmoles) de 2-[[3-[4-[[5-Méthyl-2-(1-méthyléthyl)-phénoxy]méthyl]pipéridin-1-yl]propyl]amino]pyrimidine-4-carboxylate de méthyle et 1,9 g (11,7mmoles) de (2-aminoéthyl)carbamate de 1,1-diméthyléthyle dans 15 ml d'un mélange 2/1 de propan-2-ol/méthanol, et on chauffe le mélange au reflux pendant 10h.

On évapore le solvant sous pression réduite et on purifie le résidu sur colonne d'alumine neutre (éluant : cyclohexane/-acétate d'éthyle, 80/20 à 0/100) pour obtenir 2,8 g de composé sous forme d'huile, qu'on utilise tel quel dans l'étape suivante.

4.3 *N*-(2-aminoéthyl)-2-[[3-[4-[[5-méthyl-2-(1-méthyléthyl)-phénoxylméthyl]pipéridin-1-yl]propyl] amino]-pyrimidine-4-carboxamide.

Dans un ballon de 0,25 l, on introduit 2,8 g (4,92 mmoles) de [2-[[[2-[[3-[4-[[5-Méthyl-2-(1-méthyléthyl)-phénoxy]méthyl]pipéridin-1-yl]propyl]amino]pyrimidin-4-yl]carbonyl]amino]éthyl]carbamate de 1,1-diméthyléthyle en solution dans 20 ml de dichlorométhane, puis on ajoute 20 ml d'acide trifluoroacétique et on chauffe le mélange au ref lux pendant 5h.

On évapore les solvants sous pression réduite, on ajoute au résidu brut 70 ml d'eau puis de la soude 1N. On extrait au dichlorométhane (3×150 ml) puis on lave la phase organique avec de l'eau (100 ml), on la sèche sur sulfate de sodium, on la filtre puis on évapore le solvant sous pression réduite.

On obtient 2,31 g de composé sous forme d'huile, qu'on utilise tel quel dans l'étape suivante.

4.4 *N*-[2-[[4-(aminocarbonyl)pyrimidin-2-yl]amino]éthyl]-2-[[3-[4-[[5-méthyl-2-(1-méthyléthyl)phénoxy]-méthyl]pipéridin-1-yl]propyl]amino]pyrimidine-4-carboxamide.

Dans un ballon de 0,25 l, sous atmosphère d'argon, on introduit 2,31 g (4,92 mmoles) de *N*-(2-aminoéthyl)-2-[[3-[4-[[5-méthyl-2-(1-méthyléthyl)phénoxy]méthyl]pipéridin-1-yl]propyl]amino]pyrimidine-4-carboxamide, 0,82 g (5,2 mmoles) de 2-chloropyrimidine-4-carboxamide, 0,89 g (6,4 mmoles) de carbonate de potassium et 75 ml d'acétonitrile, et on chauffe au reflux pendant 30h.
On évapore le solvant, puis on ajoute au résidu brut 100 ml d'eau et on extrait au dichlorométhane (3×100 ml). On lave la phase organique avec de l'eau (100 ml), on la sèche sur sulfate de sodium, on la filtre puis on évapore le solvant sous pression réduite. On purifie le produit par chromatographie sur colonne de gel de silice (éluant dichlorométhane/méthanol, 100/0 à 90/10) et on le recristallise dans l'acétonitrile. On isole finalement 1,75g de composé.
Point de fusion : 164-167 °C.

Exemple 5 (Composé n° 15)

(±)-*N*-[2-[[4-(aminocarbonyl)pyrimidin-2-yl]amino]éthyl]-2-[[3-[4-(5-chloro-2-méthoxyphényl) pipérazin-1-yl]-2-hydroxypropyl] amino]pyrimidine-4-carboxamide.

5.1 (±)-2-[[3-[4-(5-Chloro-2-méthoxyphényl)pipérazin-1-yl]-2-hydroxypropyl] amino]pyrimidine-4-carboxylate de méthyle.
Dans un ballon de 1 l, on introduit 4,7 g (11,16 mmoles) de (±)-2-[[3-[4-(5-chloro-2-méthoxyphényl)-pipérazin-1-yl]-2-hydroxypropyl]amino]pyrimidine-4-carboxamide et 450 ml de méthanol, puis on fait passer un courant d'acide chlorohydrique gazeux pendant quelques minutes et on chauffe à la température du reflux du méthanol pendant 2h.
On évapore le solvant sous pression réduite, on ajoute au résidu 300 ml de dichlorométhane et on refroidit à 0 °C.
On alcalinise le mélange avec une solution aqueuse saturée hydrogénocarbonate de sodium, on décante et on extrait à nouveau au dichlorométhane. On sèche la phase organique sur sulfate de sodium, on filtre et on évapore le solvant sous pression réduite. On purifie le produit par chromatographie sur colonne de gel de silice (éluant : mélange dichlorométhane/méthanol, 100/0 à 90/10) et on le recristallise dans le cyclohexane. On obtient 3,07 g d'ester.
Point de fusion : 119-122 °C.
5.2 (±)-[2-[[[2-[[3-[4-(5-Chloro-2-méthoxyphényl)pipérazin-1-yl]-2-hydroxypropyl]amino]pyrimidin-4-yl]-carbonyl]-amino]éthyl]carbamate de 1,1-diméthyléthyle.
Dans un ballon de 0,25 l, on introduit 2,21 g (5,07 mmoles) de (±)-2-[[3-[4-(5-chloro-2-méthoxyphényl)-pipérazin-1-yl]-2-hydroxypropyl]amino]pyrimidine-4-carboxylate de méthyle et 2,6 g (16,2 mmoles) de (2-aminoéthyl)carbamate de 1,1-diméthyléthyle dans 25 ml de propan-2-ol, et on chauffe le mélange réactionnel pendant 16h.
On évapore le solvant sous pression réduite et on purifie le produit brut par chromatographie sur colonne de gel de silice (éluant : dichlorométhane/méthanol, 100/0 à 90/10). On obtient 2,8 g de composé pâteux qu'on utilise tel quel dans l'étape suivante.
5.3 (±)-*N*-(2-aminoéthyl)-2-[[3-[4-(5-chloro-2-méthoxyphényl)pipérazin-1-yl]-2-hydroxypropyl]amino]-pyrimidine-4-carboxamide.
Dans un ballon de 0,5 l, on introduit 2,8 g (4,96 mmoles) de (±)-[2-[[[2-[[3-[4-(5-chloro-2-méthoxyphényl)-pipérazin-1-yl]-2-hydroxypropyl]amino]pyrimidin-4-yl]carbonyl]amino]éthyl]-carbamate de 1,1-diméthyléthyle en solution dans quelques ml de méthanol, puis, goutte à goutte, 7 ml d'acide chlorhydrique concentré. Après 15 mn d'agitation, on refroidit le mélange à 0 °C avec un mélange glace/sel/eau, puis on ajoute, par petites portions, de la soude 1N jusqu'à pH basique.
On extrait au dichlorométhane, on sèche la phase organique sur sulfate de sodium, on filtre et on évapore les solvants sous pression réduite. On obtient 2,3 g d'huile que l'on utilise telle quelle dans l'étape suivante.
5.4 (±)-*N*-[2-[[4-(aminocarbonyl)pyrimidin-2-yl]amino]éthyl]-2-[[3-[4-(5-chloro-2-méthoxyphényl)piperazin-1-yl]-2-hydroxypropyl]amino]pyrimidine-4-carboxamide.
Dans un ballon de 0,5 l sous atmosphère d'argon, on introduit 2,32 g (4,96 mmoles) de (±)-*N*-(2-aminoethyl)-2-[[3-[4-(5-chloro-2-méthoxyphényl)pipérazin-1-yl]-2-hydroxypropyl]amino]pyrimidine-4-carboxamide,0,8 g (5,1 mmoles) de 2-chloropyrimidine-4-carboxamide, 1 g (7,2 mmoles) de carbonate de potassium dans 150 ml d'acétonitrile, et on chauffe au reflux pendant 17h.
On refroidit le mélange à température ambiante, on collecte l'insoluble par filtration et on le purifie par chromatographie sur colonne de gel de silice (éluant : dichlorométhane/méthanol 100/0 à 85/15). On isole, après recristallisation dans l'acétonitrile, 0,6 g de composé.
Point de fusion : 178-181 °C.

Le tableau qui suit illustre les structures chimiques et les propriétés physiques de quelques composés selon l'invention.

**Tableau**

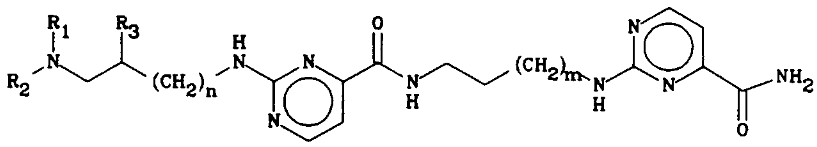

(I)

| N° | $-NR_1R_2$ | $R_3$ | n | m | Sel | F (°C) |
|---|---|---|---|---|---|---|
| 1 | 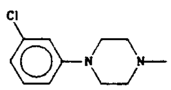 | H | 1 | 0 | -<br>fum[1] | 158-161<br>173-177 |
| 2 | 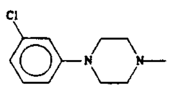 | H | 0 | 0 | -<br>HCl, $H_2O$ | 195,5-198<br>167-171 |
| 3 | 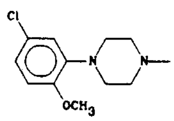 | H | 1 | 1 | - | 119-121 |
| 4 | 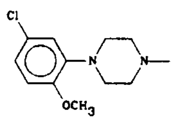 | H | 1 | 0 | HCl<br>fum | 197,5-200,5<br>175-178 |
| 5 | 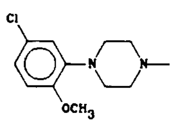 | H | 0 | 1 | - | 146-147 |
| 6 | 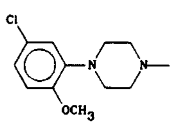 | H | 0 | 0 | - | 189-191 |
| 7 | 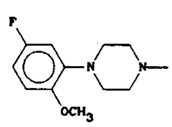 | H | 1 | 0 | - | 180-182 |
| 8 | 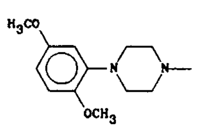 | H | 1 | 1 | - | 110-112 |

| N° | $-NR_1R_2$ | $R_3$ | n | m | Sel | F (°C) |
|---|---|---|---|---|---|---|
| 9 | 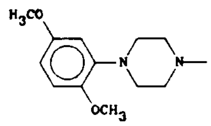 | H | 1 | 0 | - | 181-184 |
| 10 | 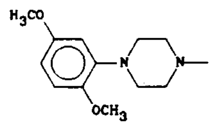 | H | 0 | 1 | - | 115,5-117,5 |
| 11 | 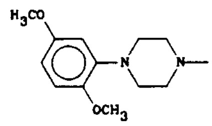 | H | 0 | 0 | - | 158-160 |
| 12 | 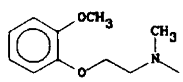 | H | 1 | 0 | - | 131-133 |
| 13 | 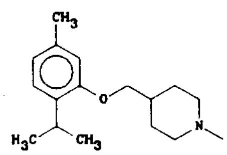 | H | 1 | 0 | - | 164-167 |
| 14 | 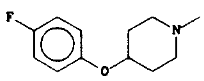 | H | 1 | 0 | - | 167-168 |
| 15 | 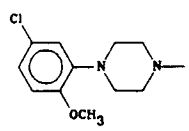 | OH | 1 | 0 | - [2] | 178-181 |
| 16 | 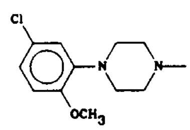 | $OCH_3$ | 1 | 0 | - [2] | 143,5-145 |

**Notes : dans la colonne "Sel", "-" désigne un composé à l'état de base, "fum" désigne un fumarate, "HCl" désigne un chlorhydrate et "$H_2O$" désigne un sel monohydraté ; [1] le composé N°1 contient 0,25 mole d'acide pour 1 mole de base ; [2] les composés N°15 et 16 sont des racémates.**

Les composés de l'invention ont fait l'objet d'études quant à leur activité antagoniste des récepteurs $\alpha$1-adrénergiques au niveau du bas appareil urinaire.

Leur activité *in vitro* a été étudiée sur l'urètre isolé de lapin.

On prépare des anneaux d'urètre de lapin mâle adulte selon la méthode de Ueda et al., *Eur. J. Pharmacol.,* (1984), **103**, 249-254, puis, après sensibilisation à la noradrénaline, on détermine la courbe concentration-réponse à la phényléphrine, en absence et en présence de composé à étudier.
On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.
Les $pA_2$ des composés sont de l'ordre de 7 à 10.

L'activité *in vivo* des composés de l'invention a été étudiée quant à leur effet sur l'hypertonie urétrale engendrée par la stimulation des fibres sympathiques du nerf hypogastrique chez le chat anesthésié.
On anesthésie des chats mâles adultes au pentobarbital sodique, et on les prépare selon la méthode de Theobald, *J. Auton. Pharmac.*, (1983), **3**, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.
On évalue la puissance de l'antagonisme $\alpha_1$-adrénergique de chaque composé par calcul de la $DI_{50}$, dose qui inhibe de 50% l 'hypertonie urétrale.
Les $DI_{50}$ des composés de l'invention sont de l'ordre de 0,01 à 1 mg/kg.

Les résultats des essais montrent que les composés de l'invention montrent, *in vitro,* une activité antagoniste des récepteurs $\alpha_1$-adrénergiques des muscles lisses du bas appareil urinaire (urètre) stimulés par un agoniste $\alpha_1$-adrénergique (phényléphrine). *In vivo*, ils inhibent l'hypertonie urétrale engendrée par la stimulation nerveuse sympathique.

Les composés de l'invention peuvent donc être utilisés pour le traitement symptomatique des maladies et affections impliquant une hyperactivité du système $\alpha$-adrénergique au niveau du bas appareil urinaire, et notamment pour le traitement des troubles mictionnels de l'hypertrophie bénigne de la prostate, tels que la dysurie et la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,1 à 500 mg de substance active.

## Revendications

1. Composé, éventuellement sous forme d'isomère optique pur ou de mélange de tels isomères, répondant à la formule générale (I)

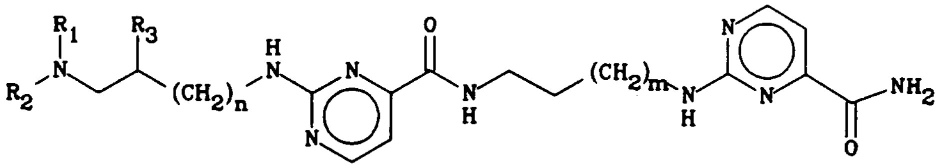

(I)

dans laquelle
n représente le nombre 0 ou 1,
m représente le nombre 0 ou 1,
$R_1$ représente un groupe méthyle, auquel cas
$R_2$ représente un groupe phénoxy($C_1$-$C_4$)alkyle (dont le groupe phénoxy porte éventuellement 1 ou 2 substituants choisis parmi les atomes d'halogènes et les groupes méthoxy), ou bien
$R_1$ et $R_2$ forment ensemble, et avec l'atome d'azote qui les porte, un groupe 4-phénoxypipéridin-1-yle (dont le groupe phénoxy porte éventuellement 1 ou 2 substituants choisis parmi les atomes d'halogènes et les groupes méthoxy), un groupe phénoxyméthylpipéridin-1-yle (dont le groupe phénoxy porte éventuellement 1 ou 2 groupes alkyles en $C_1$-$C_4$), ou un groupe 4-phénylpipérazin-1-yle (dont le groupe phényle porte éventuellement 1 ou 2 substituants choisis parmi les atomes d'halogènes et les groupes méthoxy), et
$R_3$ représente un atome d'hydrogène ou, et seulement lorsque n est égal à 1, un groupe hydroxy ou un groupe méthoxy,
à l'état de base ou de sel d'addition à une acide.

2. Composé selon la revendication 1, caractérisé en ce que $R_1$ et $R_2$ forment ensemble, et avec l'atome d'azote qui les porte, un groupe 4-phénylpipérazin-1-yle dont le groupe phényle porte 1 ou 2 substituants choisis parmi les atomes d'halogènes et les groupes méthoxy.

3. Composé selon la revendication 1, caractérisé en ce que $R_1$ et $R_2$ forment ensemble, et avec l'atome d'azote qui les porte, un groupe 4-(5-chloro-2-méthoxyphényl) pipérazin-1-yle.

4. Composé selon la revendication 1, en l'espèce le *N*-[2-[[4-(Aminocarbonyl)pyrimidin-2-yl]amino]éthyl]-2-[[3-[4-(5-chloro-2-méthoxyphényl)pipérazin-1-yl]propyl]amino]pyrimidine-4-carboxamide.

5. Procédé de préparation d'un composé selon la revendication 1, caractérisé en ce qu'on transforme un amide de formule générale (II)

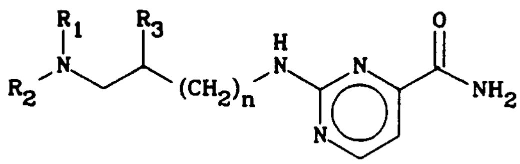

(II)

(dans laquelle n, $R_1$, $R_2$ et $R_3$ sont tels que définis dans la revendication 1) en un ester de formule générale (III)

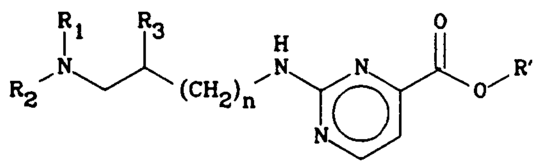

(III)

(dans laquelle R' représente un groupe alkyle en $C_1$-$C_4$), par réaction avec un alcool aliphatique en $C_1$-$C_4$, en présence d'un acide, à une température de 0 à 100 °C, puis on transforme l'ester ainsi obtenu, par réaction avec un amine de formule générale (IV)

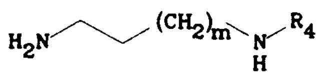

(IV)

(dans laquelle m est tel que défini dans la revendication 1 et $R_4$ représente soit un atome d'hydrogène, soit un groupement protecteur d'amine) en amide de formule générale (V)

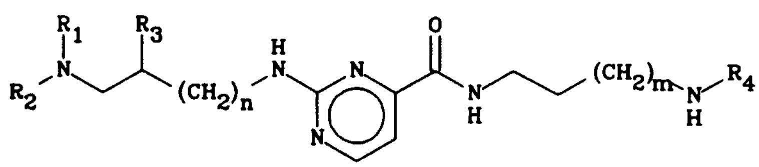

(V)

dans un alcool aliphatique comme solvant, à une température de 0 à 60 °C, puis, si nécessaire, on déprotège l'amine de formule générale (V) par une méthode connue, et enfin on fait réagir l'amine de formule générale (V) (dans laquelle $R_4$ représente un atome d'hydrogène) avec le 2-chloropyrimidine-4-carboxamide dans un solvant aprotique, en présence d'une base, à une température de 20 à 40 °C.

6. Médicament caractérisé en ce qu'il consiste en un composé selon l'une des revendications 1 à 4.

7. Composition pharmaceutique caractérisée en ce qu'elle contient un composé selon l'une des revendications 1 à 4, associé à un excipient pharmaceutique.

## Claims

1. Compound, optionally in the form of a pure optical isomer or of a mixture of such isomers, corresponding to the general formula (I)

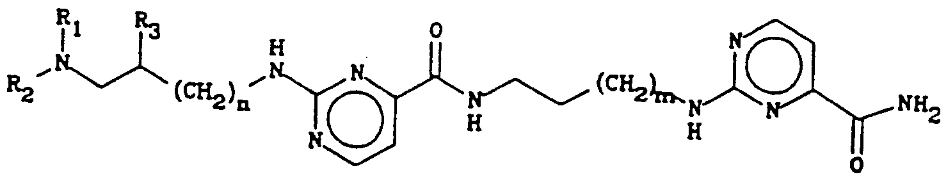 (I)

in which
n represents the number 0 or 1,
m represents the number 0 or 1,
$R_1$ represents a methyl group, in which case
$R_2$ represents a phenoxy($C_1$-$C_4$)alkyl group (in which the phenoxy group optionally carries 1 or 2 substituents chosen from halogen atoms and methoxy groups), or else
$R_1$ and $R_2$ together, and with the nitrogen atom which carries them, form a 4-phenoxypiperidin-1-yl group (in which the phenoxy group optionally carries 1 or 2 substituents chosen from halogen atoms and methoxy groups), a phenoxymethylpiperidin-1-yl group (in which the phenoxy group optionally carries 1 or 2 $C_1$-$C_4$ alkyl groups), or a 4-phenylpiperazin-1-yl group (in which the phenyl group optionally carries 1 or 2 substituents chosen from halogen atoms and methoxy groups), and
$R_3$ represents a hydrogen atom or, and only when n is equal to 1, a hydroxyl group or a methoxy group,
in the form of a base or of an addition salt with an acid.

2. Compound according to Claim 1, characterized in that $R_1$, and $R_2$ together, and with the nitrogen atom which carries them, form a 4-phenylpiperazin-1-yl group in which the phenyl group carries 1 or 2 substituents chosen from halogen atoms and methoxy groups.

3. Compound according to Claim 1, characterized in that $R_1$ and $R_2$ together, and with the nitrogen atom which carries them, form a 4-(5-chloro-2-methoxy-phenyl)piperazin-1-yl group.

4. Compound according to Claim 1, in the case in point *N*-[2-[[4-(aminocarbonyl)pyrimidin-2-yl]amino]-ethyl]-2-[[3-[4-(5-chloro-2-methoxyphenyl)piperazin-1-yl]propyl]amino]pyrimidine-4-carboxamide.

5. Process for the preparation of a compound according to Claim 1, characterized in that an amide of general formula (II)

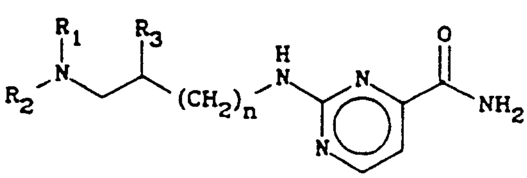 (II)

(in which n, $R_1$, $R_2$ and $R_3$ are as defined in Claim 1) is converted to an ester of general formula (III)

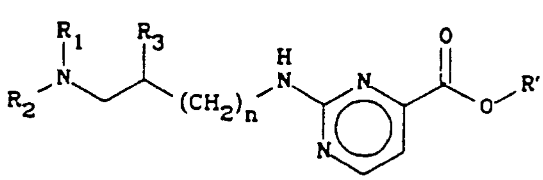 (III)

(in which R' represents a $C_1$-$C_4$ alkyl group) by reaction with a $C_1$-$C_4$ aliphatic alcohol in the presence

of an acid at a temperature of 0 to 100 ° C and the ester thus obtained is then converted, by reaction with an amine of general formula (IV)

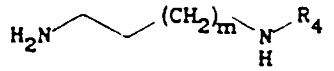 (IV)

(in which m is as defined in Claim 1 and $R_4$ represents either a hydrogen atom or an amine protective group), to an amide of general formula (V)

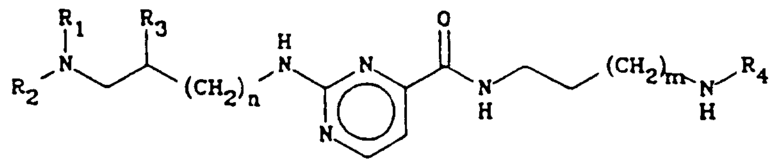 (V)

in an aliphatic alcohol as solvent, at a temperature of 0 to 60 ° C, then, if necessary, the amine of general formula (V) is deprotected by a known method and, finally, the amine of general formula (V) (in which $R_4$ represents a hydrogen atom) is reacted with 2-chloro-pyrimidine-4-carboxamide in an aprotic solvent in the presence of a base at a temperature of 20 to 40 ° C.

**6.** Medicament, characterized in that it consists of a compound according to one of Claims 1 to 4.

**7.** Pharmaceutical composition, characterized in that it contains a compound according to one of Claims 1 to 4, in combination with a pharmaceutical excipient.

**Patentansprüche**

**1.** Verbindung der allgemeinen Formel (I), die gegebenenfalls in Form des reinen optischen Isomeren oder in Form einer Mischung solcher Isomeren vorliegt:

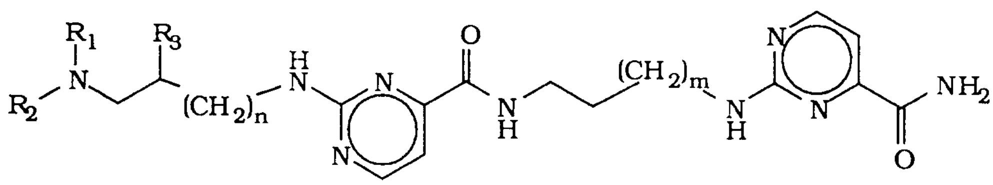 (I)

in der
n eine Zahl mit einem Wert von 0 oder 1,
m eine Zahl mit einem Wert von 0 oder 1,
$R_1$ eine Methylgruppe und in diesem Fall
$R_2$ eine Phenoxy-($C_1$-$C_4$)-alkylgruppe (deren Phenoxygruppe gegebenenfalls 1 oder 2 Substituenten ausgewählt aus Halogenatomen und Methoxygruppen trägt), oder
$R_1$ und $R_2$ gemeinsam und mit dem Stickstoffatom, das sie trägt, eine 4-Phenoxy-piperidin-1-yl-gruppe (deren Phenoxygruppe gegebenenfalls 1 oder 2 Substituenten ausgewählt aus Halogenatomen und Methoxygruppen trägt), eine Phenoxymethylpiperidin-1-yl-gruppe (deren Phenoxygruppe gegebenenfalls 1 oder 2 $C_1$-$C_4$-Alkylgruppen trägt) oder eine 4-Phenylpiperzin-1-yl-gruppe (deren Phenylgruppe gegebenenfalls 1 oder 2 Substituenten ausgewählt aus Halogenatomen und Methoxygruppen trägt) und
$R_3$ ein Wasserstoffatom oder dann, wenn n den Wert 1 besitzt, eine Hydroxylgruppe oder eine Methoxygruppe bedeuten,
in Form der Base oder des Additionssalzes mit einer Säure.

**2.** Verbindung nach Anspruch 1, **dadurch gekennzeichnet**, daß $R_1$ und $R_2$ gemeinsam mit dem Stickstoffatom, welches sie trägt, eine 4-Phenylpiperazln-1-yl-gruppe bilden, deren Phenylgruppe 1 oder 2 Substituenten ausgewählt aus Halogenatomen und Methoxygruppen trägt.

**3.** Verbindung nach Anspruch 1, **dadurch gekennzeichnet**, daß $R_1$ und $R_2$ gemeinsam mit dem Stickstoffatom, welches sie trägt, eine 4-(5-Chlor-2-methoxyphenyl)-piperazin-1-yl-gruppe bilden.

**4.** Verbindung nach Anspruch 1, nämlich N-[2-[[4-(Aminocarbonyl)-pyrimidin-2-yl]-amino]-ethyl]-2-[[3-[4-(5-chlor-2-methoxyphenyl)-piperazin-1-yl]-propyl]-amino]-pyrimidin-4-carboxamid.

**5.** Verfahren zur Herstellung einer Verbindung nach Anspruch 1, **dadurch gekennzeichnet**, daß man ein Amid der allgemeinen Formel (II)

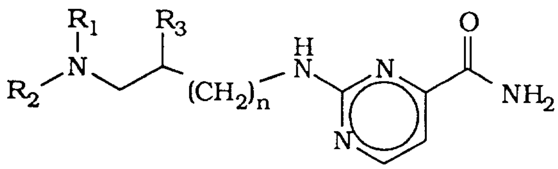

(II)

(in der n, $R_1$, $R_2$ und $R_3$ die in Anspruch 1 angegebenen Bedeutungen besitzen) durch Umsetzung mit einem aliphatischen $C_1$-$C_4$-Alkohol in Gegenwart einer Säure bei einer Temperatur von 0 bis 100 °C in einen Ester der allgemeinen Formel (III)

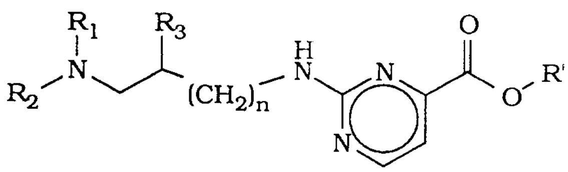

(III)

umwandelt,
(worin R' eine $C_1$-$C_4$-Alkylgruppe darstellt), dann den in dieser Weise erhaltenen Ester durch Reaktion mit einem Amin der allgemeinen Formel (IV)

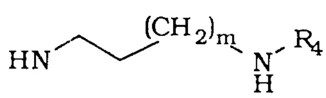

(IV)

(worin m die in Anspruch 1 angegebenen Bedeutungen besitzt und $R_4$ entweder ein Wasserstoffatom oder eine Schutzgruppe derAminogruppe darstellt) in einem aliphatischen Alkohol als Lösungsmittel bei einer Temperatur von 0 bis 60 °C in das Amid der allgemeinen Formel (V)

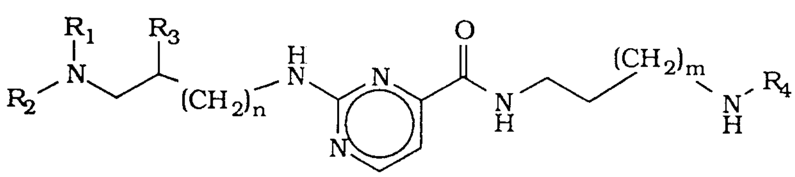

(V)

umwandelt und erforderlichenfalls mit Hilfe einer an sich bekannten Verfahrensweise die Schutzgruppe von dem Amin der allgemeinen Formel (V) abspaltet und schließlich das Amin der allgemeinen Formel (V) (worin $R_4$ ein Wasserstoffatom darstellt) mit 2-Chlorpyrimidin-4-carboxamid in einem aprotischen Lösungsmittel in Gegenwart einer Base bei einer Temperatur von 20 bis 40 °C umsetzt.

**6.** Arzneimittel, **dadurch gekennzeichnet**, daß es aus einer Verbindung nach einem der Ansprüche 1 bis 4 besteht.

**7.** Pharmazeutische Zubereitung, **dadurch gekennzeichnet**, daß sie eine Verbindung nach einem der Ansprüche 1 bis 4 in Kombination mit einem pharmazeutischen Trägermaterial enthält.